(19) Europäisches Patentamt
European Patent Office
Office européen des brevets

(11) **EP 2 199 780 A2**

(12) **EUROPÄISCHE PATENTANMELDUNG**

(43) Veröffentlichungstag:
**23.06.2010 Patentblatt 2010/25**

(51) Int Cl.:
**G01N 21/51** *(2006.01)*    **G01N 15/04** *(2006.01)*

(21) Anmeldenummer: **09158268.4**

(22) Anmeldetag: **20.04.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **21.02.2008 DE 102008010411**

(71) Anmelder: **L.U.M. GmbH**
**12489 Berlin (DE)**

(72) Erfinder:
• **Lerche, Dietmar**
  **12524 Berlin (DE)**
• **Schirg, Peter**
  **79429 Malsburg (DE)**

(74) Vertreter: **Wehlan, Helmut**
**Patentanwälte Wehlan & Wehlan**
**Möllendorffstrasse 49**
**10367 Berlin (DE)**

(54) **Verfahren und Vorrichtung zur Quantifizierung der Entmischung von Dispersionen**

(57)    Die Entmischungs- und kolloidchemische Stabilität von nano- und mikroskaligen dispersen Stoffsystemen spielt sowohl in der Entwicklung dispersionsbasierter Produkte, der Qualitätskontrolle dieser im Produktionsprozess als auch bei Separationsverfahren eine große Rolle.

Die Erfindung bestimmt die einsetzende Konzentrationsänderung (z.B. durch Entmischung) in Dispersionen an speziell ausgestalteten Grenzflächen. Die Veränderung der Konzentration wird nicht im Volumen der Probe, sondern unmittelbar an zur Migrationsrichtung der Partikel geneigten Grenzflächen mittels geeigneter konzentrationssensitiver Sensoren detektiert und der zeitliche Verlauf aufgezeichnet und bewertet.

Figur 5

EP 2 199 780 A2

**Beschreibung**

Dispersionen sind Stoffsysteme, welche aus in einer Flüssigkeit dispergierten Teilchen bestehen. Die Größe dieser Teilchen reicht von wenigen Nanometern bis zu einigen 100 Mikrometern. Die Teilchen selbst können fest (Suspension), flüssig (Emulsion) oder gasförmig (Schaum) sein. Dispersionen sind zunehmend komplizierter zusammengesetzt und die objektive und validierbare Charakterisierung der dispergierten Teilchen als auch der Gesamteigenschaften der Dispersion ist für die entsprechenden Industriezweige eine zunehmende Notwendigkeit (Literatur).

Aufgrund der in der Regel vorliegenden Dichteunterschiede zwischen der kontinuierlichen Phase und der dispergierten Phase bewirkt die Erdanziehungskraft eine Entmischung (örtliche Konzentrationsveränderung) innerhalb der Dispersionsprobe. Neben der Erdanziehungskraft können auch andere physikalische Kräfte, verursacht z.B. durch elektrische oder magnetische Felder sowie Zentrifugationsfelder eine Entmischung und damit Konzentrationsveränderungen innerhalb der Probe verursachen. Diese Entmischung kann gewollt (z.B. festflüssig Trennung) oder aber unerwünscht (z.B. langlebige Zwischen- bzw. Endprodukte, wie Agrochemikalien, Pigmentdispersionen oder Kosmetika) sein. Es besteht daher die Messaufgabe bei der Formulierung neuer Produkte, bei der Entwicklung und Optimierung von Stabilisierungsverfahren bzw. Destabilisierungsverfahren sowie im Rahmen der Qualitätskontrolle das Entmischungsverhalten zu vergleichen und zu quantifizieren. Da die Entmischungsgeschwindigkeit mit zunehmender Verbesserung der Entmischungsstabilität insbesondere bei Nanodispersionen immer kleiner wird, sind auch insbesondere Messmethoden erforderlich, welche in möglichst kurzer Zeit das Auftreten von geringsten Entmischungen durch Sedimentation oder Flotation erfassen und quantifizieren. Des Weiteren darf der Herstellungspreis einer praktischen Anwendung nicht im Wege stehen.

Das physikalische Grundphänomen im Gravitationsfeld ist dabei die auf dem Stoke's Gesetz beruhende Entmischung infolge der Erdbeschleunigung (g).

$$\varpi = \frac{2}{9} \frac{\Delta\rho \cdot x^2}{\eta} f(\alpha) \, xg \qquad\qquad Gl. \ 1$$

Die Entmischungsgeschwindigkeit ist dabei direkt von dem Dichteunterschied zwischen Partikel und Fluid ($\Delta\rho$), dem Teilchenradius (x) zum Quadrat und indirekt von der Viskosität $\eta$ des Fluids abhängig. Die hydrodynamischen Wechselwirkungen bei höheren Volumenkonzentrationen werden durch die sog. konzentrationsabhängige Hindrancefunktion $f(\alpha)$ beschrieben.

Zusätzlich ist zu berücksichtigen, dass es während der Lagerung einer Dispersion u.a. zu Größenveränderungen der Teilchen (z.B. Ostwald-Ripening, Koagulation) oder zu Strukturveränderungen (z.B. Flockung, Netzwerkausbildung) kommen kann und diese in den meisten Fällen eine Erhöhung der Entmischungsgeschwindigkeit, d.h. Verringerung der Dispersionsstabilität bewirken. Die Stabilitätsprädiktion ist daher von großem praktischem Interesse. So wurden in den letzten Jahren verschiedene Messsysteme entwickelt und auf den Markt gebracht. In der folgenden Aufstellung werden die wesentlichsten, häufig erst in den letzten 5 - 10 Jahren entwickelten Messtechniken kurz skizziert.

In der ersten Gruppe lassen sich Methoden zusammenfassen, welche die Dispersionsstabilität indirekt charakterisieren. In diesem Fall werden aus den in fragekommenden Dispersionen/Produkten Proben entnommen und man analysiert einzelne Parameter, welche im Zusammenhang mit der Dispersionsstabilität stehen. Hierzu zählen u.a. rheologische Methoden oder die Mikroskopie sowie die Bestimmung des Zeta-Potentials, der elektrophoretischen Beweglichkeit, der Oberflächenspannung oder der Partikelgröße/-verteilung.

Bekannt sind z.B. Produkte der Firmen Beckman Coulter, Brookhaven (BIC), Malvern; Dispersion Technology.

In der zweiten Gruppe sind die Methoden der direkten Bestimmung der Dispersionsstabilität zu nennen. In diesem Fall wird die entwickelte Dispersion bzw. das Produkt in eine meist zylinderförmige Messzelle verbracht und der zeitliche Verlauf der Zusammensetzung der Dispersion an verschiedenen Orten, z.B. im oberen und unteren Bereich des Probengefäßes mittels elektrischer Leitfähigkeit (vgl. DualCone, IFAC/ITEC) oder mit mechanischen Scanprozeduren mittels senkrecht zur Messzellenachse positionierten Sensoren über die Ultraschallattenuation, Lichttransmission und -streuung verfolgt (z.B. Ultrasonic Scientific, Univ. Leeds UK, oder Turbiscan, Formulaction, France).

Eine 3. Gruppe stellen die Methoden zur beschleunigten und direkten Bestimmung der Dispersionsstabilität dar. In diesem Fall erfolgt eine zeit- und ortsaufgelöste Messung von Partikel-Konzentrationsunterschieden (STEP-Technology[®]) bei einstellbarer Messzeitverringerung gegenüber der Entmischung unter Erdgravitationseinfluss (LUMi-Reader, maximal 10-fache Beschleunigung; LUMiFuge oder LUMiSizer, maximal 2300-fache Beschleunigung, alle L.U.M. GmbH, Gemany). Auch in diesem Fall sind die Lichtquelle und die entsprechende Optik sowie die bis zu 8000 Sensoren streng senkrecht zur Messzellenachse ausgerichtet (DE 4116313 C2; US6691057 B2).

Für die eingangs skizzierten Messaufgaben ist die direkte Bestimmung der Dispersionsstabilität in der Praxis von besonderem Interesse. Ohne alle methodischen Einzelheiten zu diskutieren kann man feststellen, dass sich alle bisher bekannten Analysegeräte auf die Detektion der Entmischung im Volumen der Probe möglichst weitab von den Gefäßwänden der Messzelle konzentrieren, um eine von diesen unbeeinflusste Sedimentation bzw. Flotation zu messen. Die Geräte Acousticscan, Turbiscan, LUMiReader® sowie die analytischen Zentrifugen LUMiFuge® und LUMiSizer® sind zwar in der Lage, Konzentrationsprofile entlang der Probe zu messen, jedoch nicht in der Lage kleinste Volumina, z.B. in unmittelbarer Bodennähe oder an nichtvertikalen Grenzflächen, zu analysieren. Die STEP-Technologie® der analytischen Zentrifugen verfügt auch nicht über einen Rückstreusensor, so dass die Untersuchung von Dispersionen mit hoher Volumenkonzentration in situ zusätzlich nicht gegeben ist. Ein weiterer Nachteil der bisher bekannten Lösungen ist der einen breiten Einsatz auch in kleineren Firmen entgegenstehende hohe Anschaffungspreis im Bereich von 25.000 - 75.000 Euro. Es besteht daher die Aufgabe, die skizzierten Nachteile der bisherigen Verfahren zu überwinden.

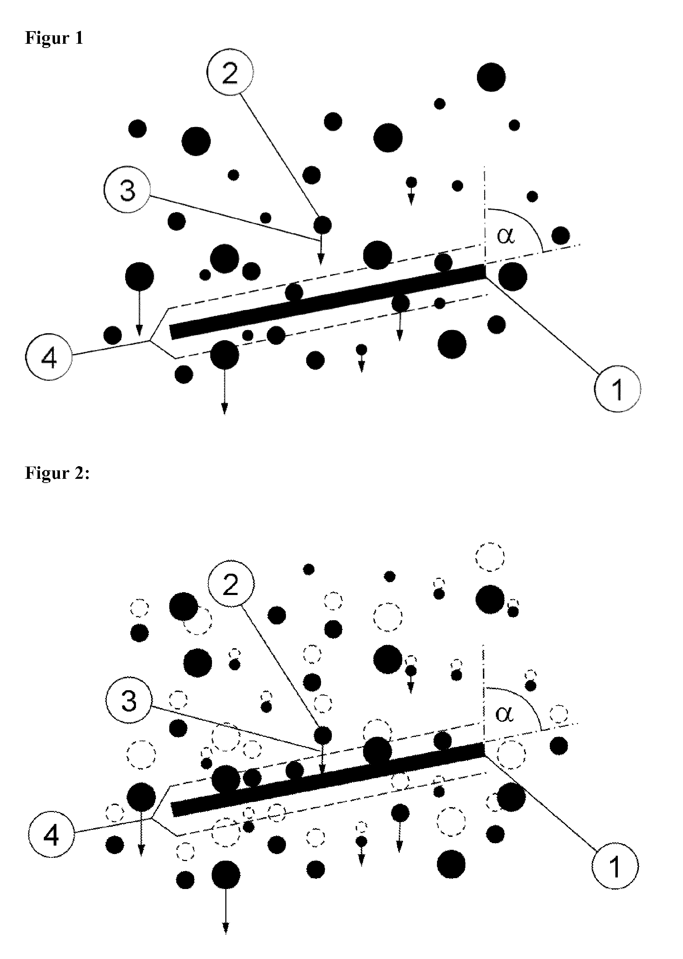

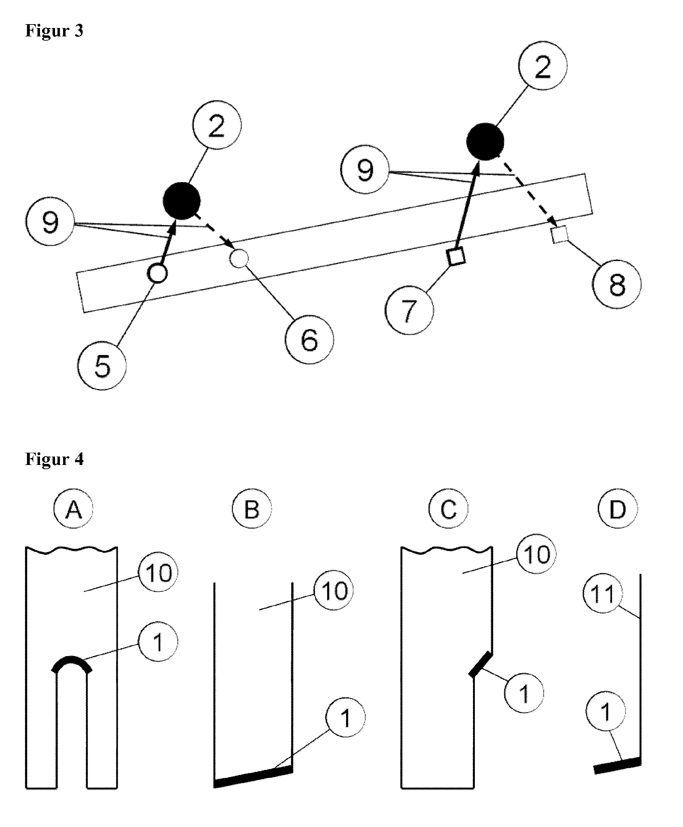

Der Erfindung liegt die Grundidee zugrunde, ein Verfahren und eine Vorrichtung zu entwickeln, welche mittels geeigneter Konzentrationssensoren und einer entsprechenden Quelle-Empfänger-Anordnung bereits <u>erste</u> einsetzende Konzentrationsveränderungen infolge von Entmischungserscheinungen bzw. sonstigen Reaktionen (z.B. chemischer Art, Kristallisation) in einer entsprechenden Dispersionsprobe in situ mit hoher Sensitivität zeitlich erfassen und in geeigneter Weise aufzeichnen kann. Die Aufgabe wurde gemäß den Merkmalen der Patentansprüche gelöst. Erfindungsgemäß kann dies am besten in unmittelbarer Nähe von zur Migrationsrichtung der Teilchen nichtparallel ausgerichteten Grenzflächen erfolgen. Die Konzentrationsveränderungen setzen hier am ersten ein und sind quantitativ am ausgeprägtesten. Figur 1 verdeutlicht schematisch eine prinzipielle Positionierung einer Grenzfläche (1) in Bezug auf die Richtung des auf die Teilchen (2) einwirkenden Kraftvektors (3). Aus Übersichtlichkeitsgründen sind nur einige Vektoren eingezeichnet. Der Positionierungswinkel beträgt in diesem Fall $\alpha$. Die Kräfte können z.B. durch Gravitations-, Zentrifugal-, elektrische oder auch magnetische Felder generiert werden und bewirken eine der Masse der Teilchen proportionale Beschleunigung. Das potentielle Messvolumen über der Grenzfläche bzw. unter der Grenzfläche ist schematisch durch die Linie 4 begrenzt. Zu Beginn des Experiments (Figur 1) sind die Teilchen homogen verteilt. Dies kann durch Schütteln der zu untersuchenden Dispersion kurz vor der Messung oder durch einen technisch realisierten Mischvorgang (z.B. Rührer) sichergestellt werden. Werden die Teilchen einer Kraft ausgesetzt, bewegen sie sich entlang der Kraftvektoren und es kommt augenblicklich sowohl unmittelbar über als auch unmittelbar unter der Grenzfläche zu einer Konzentrationsveränderung (Figur 2). Dabei stellen die offenen Kreise die Teilchen in Ausgangsposition dar (t = 0) und die vollen Kreise die Positionen nach der Wanderungszeit $t = t_1$. Überraschend verdoppelt sich die Konzentration der Teilchen über der Grenzfläche bzw. sinkt sogar auf Null unter Grenzfläche, wenn durch den Kraftvektor die Teilchen lediglich einen Weg, welcher dem Abstand der das Messvolumen begrenzenden Linie 4 (in Figur 1 und 4) entspricht, gewandert sind, während außerhalb des Messvolumens, d.h. im Dispersionsvolumen in einiger Entfernung von der Grenzfläche, es zu keinen Konzentrationsveränderungen kommt. Erfindungsgemäß können die einsetzenden Konzentrationsveränderungen im Messvolumen mit einem oder mehreren Konzentrationssensoren gemessen werden. Besonders vorteilhaft ist die berührungslose Erfassung z.B. mittels elektromagnetischer Wellen. Figur 3 zeigt zwei mögliche Ausführungen, basierend auf dem optischen Rückstreuverfahren. Die Lichtquelle (5) und der Rückstreuempfänger (6) sind im einen Fall in die Grenzfläche integriert und im zweiten Fall unterhalb der Grenzfläche (Quelle 7 und Empfänger 8) angeordnet. Um die Übersichtlichkeit zu wahren, wurde der Strahlengang 9 schematisch für jeweils nur ein Streuteilchen (2) eingezeichnet. Es sind aber auch andere Messverfahren, wie die Erfassung der Konzentration durch die Messung der optischen Transmission oder der Schallausbreitungsgeschwindigkeit/Schallschwächung parallel zur Grenzfläche möglich. Auch kann mittels Oberflächenelektroden, welche in die Oberfläche der Grenzfläche integriert sind, die Konzentration erfasst werden. In diesem Fall kann besonders einfach und vorteilhaft im Sinne von Figur 2 die Sensitivität der Konzentrationsmessung verdoppelt werden, indem die Messung sowohl an der oberen und an der unteren Grenzfläche erfolgt und beide Messsignale in Kombination ausgewertet werden.

Erfindungsgemäß kann die Grenzfläche als separate Vorrichtung an einer beliebigen Stelle des die Dispersionsprobe aufnehmenden Probengefäßes oder des die Dispersion enthaltenen Behältnisses platziert werden oder aber vorteilhaft als Teil des Probenaufnahmegefäßes, z.B. als Boden, ausgebildet werden. Die letztere Ausgestaltung hat zusätzlich den Vorteil, dass für die Messung nur sehr kleine Probenvolumina benötigt werden, was insbesondere in der Entwicklung von neuen Wertstoffen von Bedeutung ist. Dabei kann die Grenzfläche in Abhängigkeit von den realen Gegebenheiten (Dispersion) und der Ausgestaltung des/der Konzentrationssensoren sowohl eben als auch gekrümmt sein, womit gleichzeitig auch eine Optimierung der Sensoreigenschaften ermöglicht wird. Genannt sei hier exemplarisch die Minimierung von das Messsignal störenden Reflexionen am Grenzflächenmaterial bei der Verwendung von unter der Grenzfläche positionierter Sender-Empfänger-Anordnung (Figur 3). Vorteilhaft kann weiterhin die Verwendung einer Wellenlänge im NIR-Bereich sein, da hiermit Einflüsse des Tageslichtes minimiert werden können.

Überraschend hat sich gezeigt, dass durch unterschiedliche Neigungswinkel $\alpha$ der Grenzfläche mechanische Eigenschaften des sich bildenden Sediments, wie Fliessen oder Sticking erfasst werden können. In diesem Fall ist eine sphärische bzw. asphärische Krümmung von Vorteil, da bei einem kritischen minimalen Winkel das Fliessen zum Stillstand kommt.

Die von den Konzentrationssensoren aufgenommenen analogen Spannungssignale sind direkt oder digitalisiert als Funktion der Messzeit zu speichern oder in geeigneter Weise aufzuzeichnen. Dabei erweist es sich als vorteilhaft, wenn das Signal bezüglich physikalischer Referenzen (max. und min. Signalhöhe) abgeglichen wird, um eine Vergleichbarkeit unterschiedlicher Proben zu ermöglichen. Es hat sich auch gezeigt, dass eine online Datenverarbeitung sinnvoll ist und der Entmischungsprozess durch einfache Zeitfunktionen, wie

$$\text{Signal} = A(1 - \exp(B\ t^{K})) + C \qquad\qquad \text{Gl. 2}$$

oder

$$\text{Signal} = A(1 - \exp(B\ t^{K}))\ \exp(D\ t) + C \qquad\qquad \text{Gl. 3}$$

sich beschreiben lässt, wobei A,B,C und D jeweils Geräte- bzw. probenabhängige Konstanten sind und t für die Zeit steht.

Die Primärverarbeitung des zeitlichen Verlaufs des Messsignals erfolgt automatisch über Algorithmen mittels der Firmware im Mikrocontroller. Die Konstanten entsprechend Gl. 2 und Gl. 3 werden als probenspezifische Kenngrößen mit dem zu Beginn des Experiments gespeicherten Probennamen, dem Datum, der Messzeit und dem verwendeten Auswertealgorithmus über ein Display und/oder wahlweise eine Schnittstelle ausgeben.

Überraschend hat sich gezeigt, dass die Auswertung des Vorzeichens der Änderung des Signals eine schnelle und einfache Möglichkeit zur Klassifizierung von Entmischungserscheinungen ermöglicht. Zur prinzipiellen Erklärung sei von einer in das Probengefäß integrierten Grenzfläche als Boden (vgl. auch Ausführungsbeispiel 1, Figur 4B) ausgegangen. So handelt es sich bei der Zunahme immer um solche Entmischungsprozesse, welche in Summe zu einer Zunahme der Konzentration der Teilchen im unmittelbaren Messvolumen über dem Boden führen. Mit anderen Worten die Teilchen sedimentieren. Im entgegengesetzten Fall wird die Entmischung bzw. Instabilität durch Flotationserscheinungen hervorgerufen. Dieses Erfindungsmerkmal ermöglicht es in effektiver Weise z.B. den Inversionspunkt von einer Ölin-Wasser-Emulsion in eine Wasser-in-Öl-Emulsion z.B. in Abhängigkeit von den Volumenanteilen, Additiven oder der Temperatur zu bestimmen. Besonders viel versprechend ist die Ausnutzung dieses Erfindungsmerkmals für die einfache und schnelle Bestimmung der Dichte von dispergierten Teilchen. Im Fall, dass die Teilchen eine geringere Dichte haben, kommt es zur Abnahme des Signals und umgekehrt. Erfindungsgemäß kann die obige Aufgabe durch den Einsatz einer Multiprobengefäßeinheit am effektivsten gelöst werden. Erfindungsgemäß kann die Messzelle auch temperiert werden, damit kann z.B. auch der Einfluss der Temperatur auf Inversionsphänomene bzw. die Stabilität der Dispersion untersucht werden.

Die Primärverarbeitung des zeitlichen Verlaufs des Messsignals erfolgt über zu entwickelnde Algorithmen automatisch mittels der Firmware im Mikrocontroller. Geeignete Kenngrößen sind mit dem Probennamen, dem Datum, der Messzeit und dem verwendeten Auswertealgorithmus über ein Display und/oder wahlweise eine Schnittstelle auszugeben.

**Ausführungsbeispiele:**

1. Beispiel

Die Anordnung der Grenzfläche (1) integriert in das Probengefäß (10) ist in Figur 4A, 4B und 4C schematisch dargestellt, ohne das damit alle Realisierungen aufgezeigt werden. Des Weiteren ist in Figur 4D die Ausgestaltung der Erfindung in einer vom Probengefäß unabhängigen Variante schematisch dargestellt. In diesem Fall ist die Grenzfläche (1) mechanisch an einem Halter (11) befestigt und kann damit frei in der in einem Behältnis gelagerten Dispersion positioniert werden und es entfallen die Probenentnahme sowie deren Verbringung in das Probengefäß. Auch kann durch die Ausrichtung des Halters (11) bezüglich der Migrationsrichtung eine Signaloptimierung einfach realisiert werden.

2. Beispiel

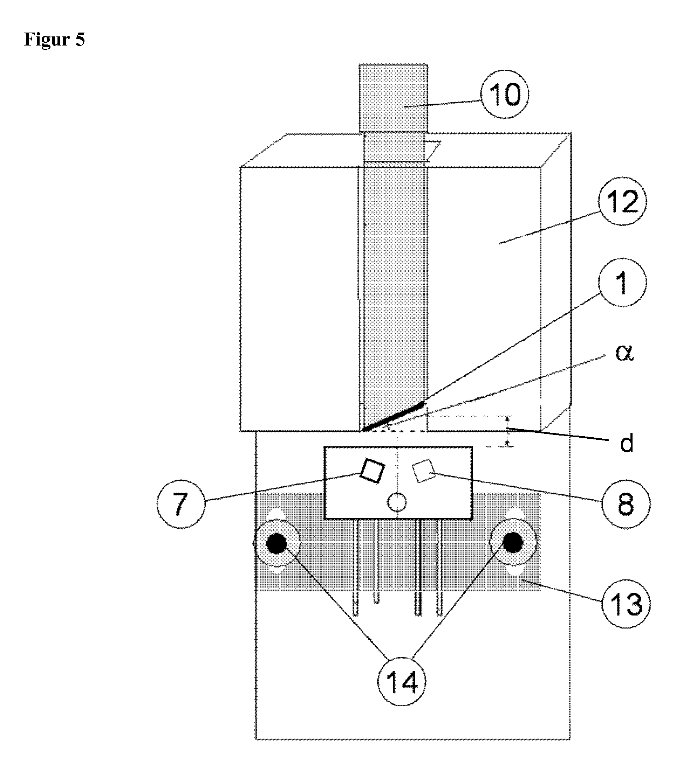

Figur 5 zeigt den schematischen Versuchsaufbau für die Charakterisierung einer Polystyrendispersion, bestehend aus dem rechteckigen Probengefäß (10) mit einer Querschnittsfläche von 10x10 mm$^2$, dem Probengefäßhalter (12) und einer NIR-LED vom Typ SFH 4503 (13) sowie dem Empfänger vom Typ SFH205 F (14). Quelle und Empfänger sind auf eine Halteplatte (15) befestigt, welche mittels zweier Schrauben (16) sowohl bezüglich des Abstandes d und der Ausrichtung zur Grenzfläche (1) justiert und befestigt werden. Durch Optimierung des Abstandes d kann z.B. auch die Größe des erfassten Messvolumens z.B. an die Konzentration der Messprobe angepasst werden.

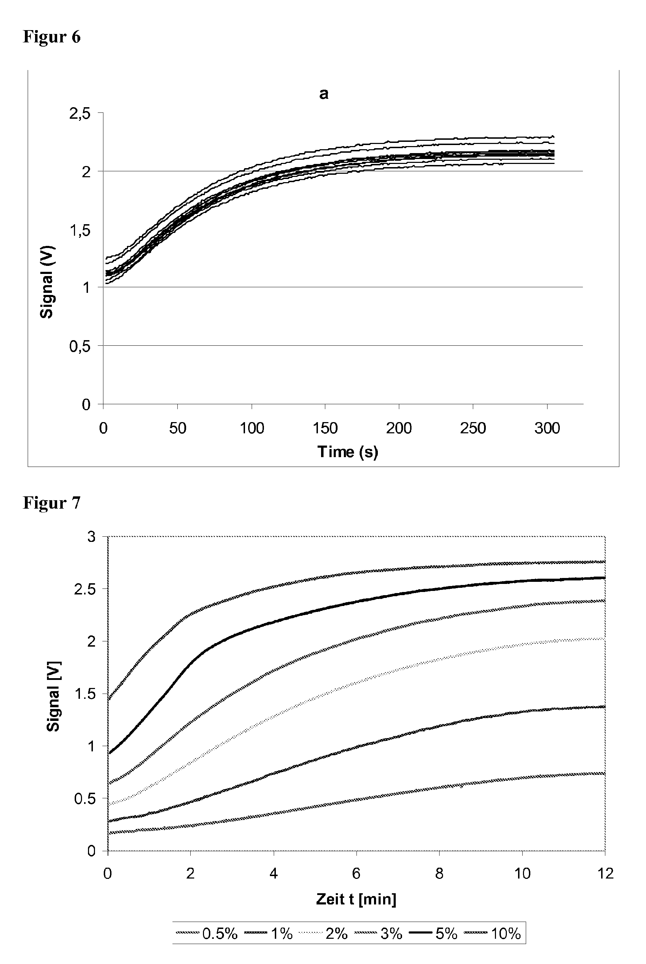

Dynoseeds TS 80 von Microbeads AS wurden in einer wässrigen 0,15% m/m Lösung von Sodium Dodecylsulfate (SDS) aufgeschlämmt, gut geschüttelt und 5 ml in das Probengefäß (10) verbracht. Figur 6 zeigt das Empfängerausgangssignal in Abhängigkeit von der Zeit (t = 0, Einfüllzeitpunkt) für 10 Experimente der gleichen Probe.

3. Beispiel:

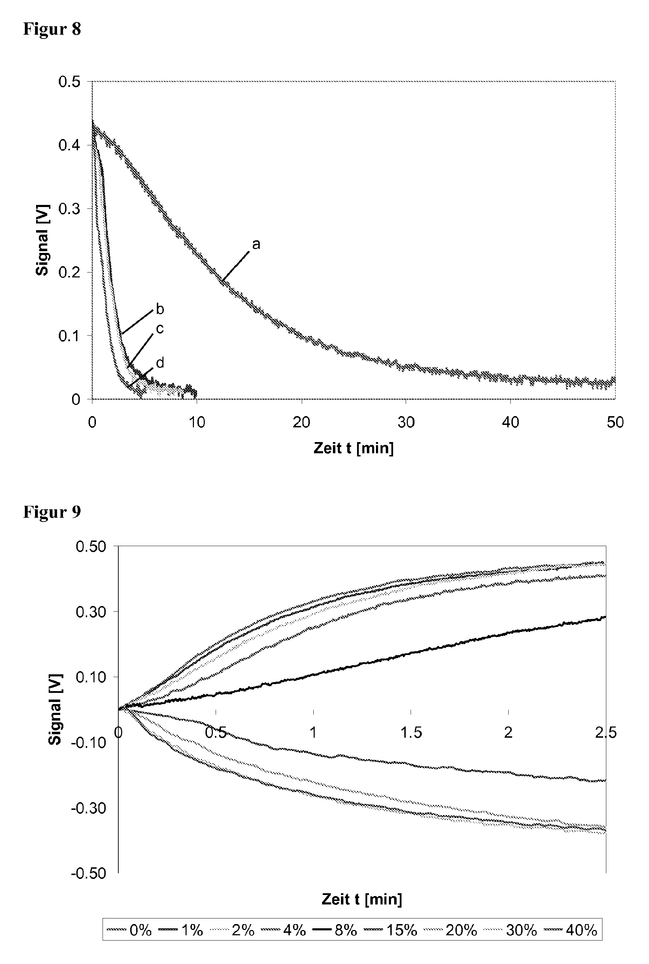

Im folgenden sollen zwei ausgewählte Anwendungsbeispiele gezeigt werden. Es wurde ein zu Beispiel 5 weitgehend analoger Versuchsaufbau für alle Messungen gewählt. Figur 7 zeigt den Einfluss der Ausgangsmassenkonzentration auf das Messsignal bei einer Grenzflächenneigung von 5 ° für eine Entmischung durch Sedimentation (15 $\mu$m PMMA Particle, Einfüllhöhe 2 cm). Diese Messbeispiel belegt für t = 0 deutlich, dass entsprechend der Erfindung die Höhe des Sensorsignals durch die Konzentration der Teilchen im Messvolumen bestimmt wird. In Figur 8 ist ein Ausführungsbeispiel für die Entmischung einer Emulsion (6,8 % Parafin Oil in 1,17 molarer NaCl-Lösung) gezeigt. Kurve a zeigt das entsprechende Entmischungssignal für die stabile Emulsion. Infolge der Aufrahmung (Verminderung der Konzentration im Messvolumen über der Grenzfläche) sinkt das Rückstreusignal und zeigt deutlich eine flotierende Entmischung an. Interessanterweise verhält sich eine Wasser-in-ÖL-Emulsion genau umgekehrt und das Verfahren ermöglicht damit die einfache Bestimmung des Inversionspunktes. Wie man auf Figur 8 weiter sehen kann, führt die Destabilisierung der Emulsion (starkes Schütteln vor dem Versuch führt zur Flockung) zu einem deutlich veränderten Messsignal (Kurven b, c, d).

4. Beispiel

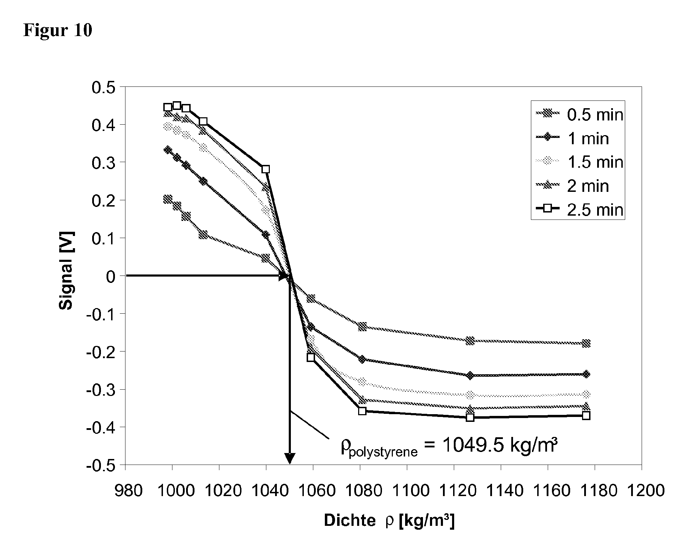

Entsprechend Gl. 1 kann man die Entmischungsgeschwindigkeit durch die Veränderung de Viskosität des Fluids und des Dichteunterschieds beeinflussen. Figur 9 zeigt den Einfluss der Zuckerkonzentration auf die Entmischung von Polystyrene Powder (80 $\mu$m, 10 % m/m). Ohne Zuckerzusatz ist die zeitliche Veränderung am ausgeprägtesten (obere Kurve) und verringert sich mit zunehmender Konzentration. Da die Veränderung der Zuckerkonzentration nicht nur die Viskosität sondern auch die Dichte der Lösung erhöht, kommt es bei einer Konzentration zwischen 8 % und 15 % zur Umkehr der Migrationsrichtung, da nun die Dichte der Teilchen kleiner als die der Lösung ist. Wie weiter oben dargestellt, kann damit die Erfindung auch zur Bestimmung der Dichte von Teilchen genutzt werden. Figur 10 belegt, dass die Dichte in Einfacherweise zu 1049,6 Kg/m$^3$ bestimmt werden kann, was sehr gut mit den Herstellerangaben von 1050 kg/m$^3$ übereinstimmt.

**Patentansprüche**

1. Verfahren zur Charakterisierung von verdünnten bzw. konzentrierten Dispersionen mittels Sensoren, **gekennzeichnet dadurch, dass** in einer oder mehreren Dispersionsproben deren Konzentrationsveränderungen infolge physikalischer Kräfte und/oder anderer Reaktionen über oder unter an zur Migrationsrichtung der Dispersionsteilchen nichtparallelen Grenzflächen mittels eines oder mehrerer konzentrationsempfindlicher Sensoren oder Messsystem(s)e detektiert und deren zeitlicher Verlauf quantitativ und/oder qualitativ bewertet wird.

2. Verfahren nach Anspruch 1, **gekennzeichnet dadurch, dass** die Grenzflächen

   a) eben, sphärisch oder asphärisch ausgebildet sind und / oder
   b) als Teil eines Messgefäßes, z.B. als Boden, ausgebildet ist oder als separate Einheit in der Probe platziert wird.

3. Verfahren nach Anspruch 1 oder 2, **gekennzeichnet dadurch, dass** die Konzentrationsbestimmung in unmittelbarer Grenzflächennähe durch Sensoren, welche in die Grenzflächen integriert, an bzw. auf den Grenzflächen angebracht sind oder berührungslos durch Messsysteme basierend auf transmittierenden oder reflektierenden Wellen erfolgt.

4. Verfahren nach einem der Ansprüche 1 bis 3, **gekennzeichnet dadurch, dass** unter dem vorzugsweise 1° - 10 ° gegen die vertikale geneigten, transparenten Boden des Messgefäßes eine Lichtquelle und ein entsprechender Detektor im Sinne einer Reflexlichtschranke so angeordnet sind, dass die sich ändernde Partikelkonzentration unmittelbar über dem Gefäßboden eine maximale Veränderung des Detektorsignals ergibt.

5. Verfahren nach Anspruch 4, **gekennzeichnet dadurch, dass** durch eine Veränderung der Position bzw. der Ausrichtung des Senders und/oder des Empfängers das erfassbare Messprobenvolumen der Dispersionskonzentration angepasst werden kann.

6. Verfahren nach einem der Ansprüche 1 bis 5, **gekennzeichnet dadurch, dass**

   a) die Messprobe und/oder das gesamte Messsystem temperiert wird und / oder
   b) die Messprobe vor Start der Messung durch eine geeignete Vorrichtung gemischt wird.

7. Verfahren nach einem der Ansprüche 1 bis 6, **gekennzeichnet dadurch, dass** das Detektorsignal kontinuierlich oder in wählbaren Zeitintervallen aufgezeichnet, das Entmischungsverhalten im Falle der Abnahme als Flotation und im Falle der Zunahme als Sedimentation klassifiziert sowie der Betrag des Messsignals in Relation zu einem schwarzen Körper mit einem Reflektionsgrad von "Null %" und einen weißen Streustandard mit einem Reflektionsgrad von "100 %" gesetzt und aus dem Verlauf des normierten Messsignals durch Integration bzw. Ermittlung entsprechender Fitfunktionen das Entmischungsverhalten quantitativ beschrieben wird.

8. Verfahren nach einem der Ansprüche 1 bis 7, **gekennzeichnet dadurch, dass** durch Dispergieren von Teilchen mit unbekannter Dichte in Medien mit unterschiedlicher Dichte, die Dichte der Teilchen der Dichte des Mediums entspricht, für welche es zu keiner Signalveränderung über die Zeit kommt oder dieser Dichtewert durch die Auftragung der Signaländerung pro Zeiteinheit gegen die Dichte des Mediums durch Interpolation gewonnen wird.

9. Vorrichtung zur Charakterisierung von verdünnten bzw. konzentrierten Dispersionen, umfassend eine µC-gesteuerten Multiprobenaufnahmeeinheit mit Probenaufnahmegefäßen und Datenlogger zur Aufzeichnung von Messsignalen, verbunden mit einem Auswertemodul, einem µC oder PC zur Datenanalyse, **dadurch gekennzeichnet, dass** die Probenaufnahmegefäße

   a) mit zur Migrationsrichtung (3) von Dispersionsteilchen (2) nichtparallelen Grenzflächen (1) ausgestattet sind und
   b) einen oder mehrere Sensoren zur Erfassung der Konzentration unmittelbar über und/oder unter der Grenzfläche besitzen.

10. Vorrichtung nach Anspruch 9 **gekennzeichnet dadurch, dass** die Grenzfläche (1)

    a) integraler Bestandteil des Probengefäßes ist und / oder
    b) eben oder gekrümmt ist und / oder
    c) mittels eines Halters an eine beliebige Stelle im Probengefäß platziert wird und / oder
    d) temperiert werden kann.

11. Vorrichtung nach Anspruch 9 oder 10, **gekennzeichnet dadurch, dass** vorzugsweise Sensoren mit optisch-, akustisch-, leitfähigkeits- oder potentialbasierten Messprinzipien eingesetzt werden und diese über und/oder unter der Grenzfläche platziert und/oder in die Grenzfläche integriert werden.

12. Vorrichtung nach einem der Ansprüche 9 bis 11, **gekennzeichnet dadurch, dass**

    a) mehrere Probengefäße mit den gleichen Konzentrationssensoren oder mit unterschiedlichen Konzentrationssensoren in eine Vorrichtung integriert werden und / oder
    b) das Probengefäß mit integrierter Grenzfläche oder mit durch einen Halter beliebig positionierter Grenzfläche auf einen Zentrifugenrotor platziert ist und das Zentrifugalfeld nicht parallel zur Grenzfläche gerichtet ist und / oder
    c) die Probengefäße als Einweggefäße ausgestaltet sind und / oder
    d) in das bzw. die Probengefäße eine Mixvorrichtung integriert ist.

13. Vorrichtung nach einem der Ansprüche 9 bis 12, **gekennzeichnet dadurch, dass** die Ausrichtung der Grenzfläche über eine Justiervorrichtung bezüglich der Migrationsrichtung der Teilchen veränderbar ist.

14. Vorrichtung nach Anspruch 9, **gekennzeichnet dadurch, dass** die Dispersion mit einem zusätzlichem auf die Teilchen einwirkenden Kraftfeld vorzugsweise einem elektrischen oder magnetischen Feld beaufschlagt werden kann.

15. Vorrichtung nach 9, **gekennzeichnet dadurch, dass** die Messgefäße automatisch mittels z.B. eines Roboters in die Vorrichtung platziert und aus ihr entnommen oder gefüllt, entleert und gereinigt werden und der Messvorgang nach dem Plazieren oder Füllen sowie gegebenenfalls dem Mischen automatisch gestartet wird.

**Figur 1**

**Figur 2:**

**Figur 3**

**Figur 4**

**Figur 5**

**Figur 6**

**Figur 7**

**Figur 8**

**Figur 9**

**Figur 10**

$\rho_{polystyrene}$ = 1049.5 kg/m³

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4116313 C2

- US 6691057 B2